# EUROPEAN PATENT SPECIFICATION

(11) **EP 1 155 679 B1**
(45) Date of publication and mention of the grant of the patent: **28.02.2007**
(21) Application number: 01110127.6
(22) Date of filing: 03.05.2001
(51) Int. Cl.: A61Q 5/10, A61K 8/23, A61K 8/67, A61K 8/41, A61K 8/34, A61K 8/49

(54) **Hair dye**
Haarfärbemittel
Teinture pour cheveux

(30) Priority: 11.05.2000 JP 2000138468
(43) Date of publication of application: 21.11.2001
(73) Proprietor: KAO CORPORATION, Tokyo 103-8210 (JP)
(72) Inventor: Matsunaga, Kenichi, c/o Kao Corporation, Tokyo 131-8501 (JP); Miyabe, Hajime, c/o Kao Corporation, Tokyo 131-8501 (JP)
(74) Representative: HOFFMANN EITLE

(56) References cited:
- EP-A- 0 739 622
- EP-A- 0 998 908
- FR-A- 2 773 471
- US-A- 5 993 490

## Description

### Technical Field

The present invention relates to a hair dye containing an oxidation dye and, in combination therewith, a cationic direct dye. More specifically, the invention pertains to a hair dye which is capable of dyeing the hair in a vivid yellow to green or orange color tone, has high color fastness and is free of color disappearance of the direct dye in the stage of storage.

### Background Art

As a typical hair dye, known is a two-part permanent hair dye composed of a first component part containing an alkali agent, an oxidation dye and the like and a second component part containing an oxidizing agent. Since the color tone produced by the oxidation dye is not so vivid, it is the common practice to use a direct dye in combination therewith in order to realize a color shade which is not attainable only by this oxidation dye. Particularly it is important to assist the oxidation dye to produce a yellow color, which cannot be easily attained only by it, by using a direct dye.

Nitrobenzene dyes are known as a typical direct dye to be used in combination with an oxidation dye for such a purpose. Nitrobenzene dyes are however accompanied with the drawback that in spite of a vivid color immediately after dyeing, color fastness to shampooing is low and therefore, the color produced by them does not last long. In order to improve color fastness, combined use of a direct dye having a larger molecular size has been proposed. Moreover, to overcome deterioration in water solubility due to an increase in the molecular size, use of, as a direct dye, a cationic compound having a quaternary nitrogen atom has been proposed.

Many of the above-described direct dyes however involve the problem that their color disappears gradually owing to their reaction with a reducing agent added for preventing too early oxidation of an oxidation dye precursor in the stage of storage before use.

EP 0 998 908 A refers to a composition for the oxidation dyeing of keratin fibers which include as an oxidation dye precursor a pyrazole-[1,5-a]-pyrimidine and a cationic direct dye. The composition further comprises a coupler which may be represented by a meta-amino phenol, metaphenylene diamine, metadiphenol or a heterocyclic coupler. The composition may include an alkali agent and its pH-value is between 3 to 12, preferably between 5 to 11.

In FR 2 773 471 A, an oxidation dye composition for keratinous fibers is disclosed which comprises an oxidation dye precursor, a cationic direct dye and a specific enzyme. The oxidation colours can comprise an oxidation dye precursor and couplers. The couplers may include meta-phenylene diamines, metaamino phenols, metadiphenols, and heterocyclic couplers including acid addition salts thereof. The dyeing composition is based in water or a mixture of water with an organic solvent, and the pH-value of the composition is between 4 and 11.

US 5 993 490 A reveals a ready-to-use-composition for the oxidation dyeing of keratin fibers comprising an oxidation base being paraphenylenediamine, a bis(phenyl)alkylenediamine or an acid addition salt thereof, at least one coupler being metadiphenol or an acid addition salt thereof, an oxidizing agent and a cationic direct dye. The medium suitable for dyeing comprises water or a mixture of water and at least one organic solvent. The pH-range is from 5 to 12.

EP 0 739 622 A concerns a composition for the oxidation dyeing of hairs including an azo dye, a coupler, a reduction agent and an oxidation agent.

### Disclosure of the Invention

An object of the present invention is therefore to provide a hair dye which is capable of dyeing the hair in a vivid color tone, has high color fastness and is free of disappearance of the color of the direct dye in the stage of storage.

The present inventors have found that upon use of a cationic direct dye in combination with an oxidation dye, a hair dye which is capable of dyeing the hair in a vivid, yellow to green or orange color, has high color fastness and is free of disappearance of the color of the direct dye in the stage of storage is available by using a cationic dye represented by the below-described formula (1) together with a blue-color-producing coupler such as meta-diaminobenzene or pyridine type coupler or a red-color-producing coupler such as meta-aminophenol, naphthol or pyridine type coupler.

In the present invention, there is thus provided a hair dye comprising a first component part containing an oxidation dye precursor and a second component part containing an oxidizing agent, wherein the first component part contains (A) a precursor, (B) a meta-diaminobenzene, meta-aminophenol, pyridine or naphthol type coupler, (C) a cationic direct dye represented by the following formula (1): 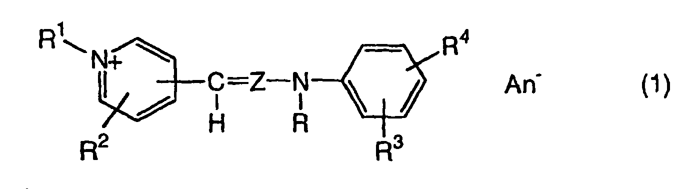 [wherein, R¹ represents a C₁₋₄ alkyl group which may have a hydroxy or cyano group as a substituent, R² represents a hydrogen atom, a C₁₋₄ alkyl group or a cyano group, Z represents a nitrogen atom or a methine group, R represents a hydrogen atom or a C₁₋₄ alkyl group, R³ and R⁴ each independently represents a hydrogen atom, a C₁₋₄ alkyl group, C₁₋₄ alkoxy group, an amino group or a halogen atom, or R and R³ may form a 5- or 6-membered ring together with the adjacent nitrogen atom and the carbon atom on the benzene ring, and An⁻ represents an anion], (D) an alkali agent, (E) a reducing agent and (F) water; and has a pH of 8 to 12.

### Best Modes for Carrying Out the Invention

Examples of the precursor to be incorporated in the first component part as component (A) include para-phenylenediamine, toluene-2,5-diamine, 2-chloro-para-phenylenediamine, N-methoxyethyl-para-phenylenediamine, N,N-bis(2-hydroxyethyl)-para-phenylenediamine, 2-(2-hydroxyethyl)-para-phenylenediamine, 2,6-dimethyl-para-phenylenediamine, 4,4'-diaminodiphenylamine, 1,3-bis(N-(2-hydroxyethyl)-N-(4-aminophenyl)amino)-2-propanol, PEG-3,2,2'-para-phenylenediamine, para-aminophenol, para-methylaminophenol, 3-methyl-4-aminophenol, 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine and 4,5-diamino-1-(4'-chlorobenzyl)pyrazole, and salts thereof.

These precursors serving as component (A) may be used either singly or in combination. Its (their) content is preferably 0.1 to 5 wt.%, especially 0.5 to 3 wt.% based on the whole composition (after mixture of the first and second component parts, which will equally apply hereinafter).

Examples of the coupler to be incorporated in the first component part as component (B) include meta-diaminobenzene type such as meta-phenylenediamine, 2,4-diaminophenoxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene and 1,3-bis(2,4-diaminophenoxy)propane, and salts thereof; meta-aminophenol type ones such as meta-aminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-meta-aminophenol, 2-methyl-4-methoxy-5-(2-hydroxyethylamino)phenol and 2-methyl-4-fluoro-5-aminophenol and salts thereof; pyridine type ones such as 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine and 2,6-diaminopyridine, and salts thereof; and naphthol type ones such as 1-naphthol, 2-naphthol, 1,5-dihydroxynaphthalene, 1,7-dihydroxynaphthalene and 2,7-dihydroxynaphthalene, and salts thereof.

These meta-diaminobenzene, meta-aminophenol, pyridine and naphthol type couplers serving as component (B) may be used either singly or in combination. Its (or their) content is preferably 0.1 to 5 wt.%, especially 0.5 to 3 wt.% based on the whole composition.

In the cationic direct dye to be incorporated in the first component part as component (C), the C₁₋₄ alkyl groups represented by R, R¹, R², R³ or R⁴ in the formula (1) are, for example, methyl, ethyl, propyl, butyl and isopropyl groups. Of these, methyl and ethyl groups are preferred. Examples of the substituted alkyl group represented by R¹ include 2-hydroxyethyl and 2-cyanoethyl groups.

Examples of the C₁₋₄ alkoxy group represented by R³ or R⁴ include methoxy, ethoxy, propoxy and isopropoxy groups. Of these, methoxy and ethoxy groups are preferred.

Examples of the anion represented by An⁻ include chloride, bromide, iodide, trichlorozincic acid, tetrachlorozincic acid, sulfuric acid, hydrosulfuric acid, methyl sulfate, phosphoric acid, formic acid and acetic acid ions.

The followings are specific examples of the cationic direct dye (1) of the component (C).

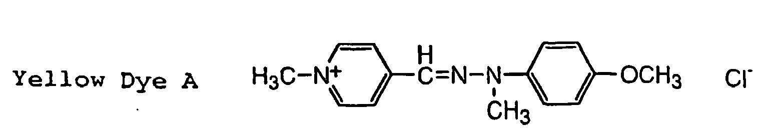

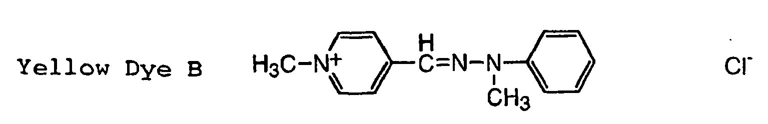

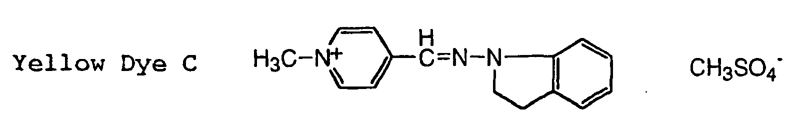

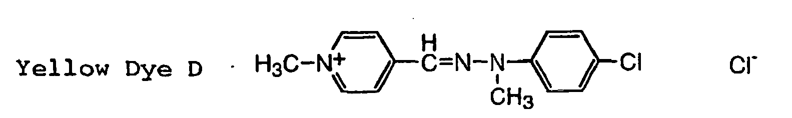

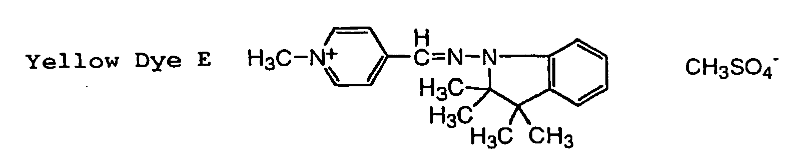

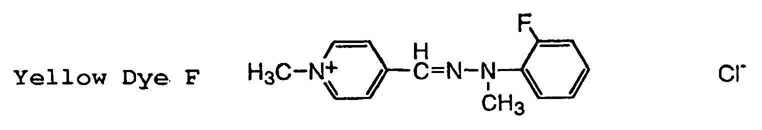

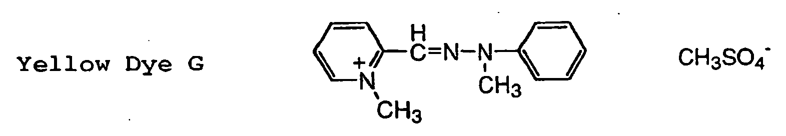

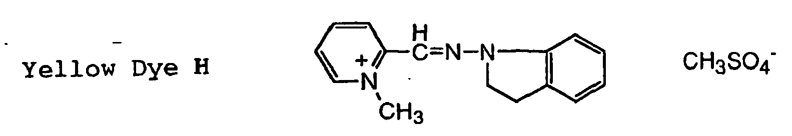

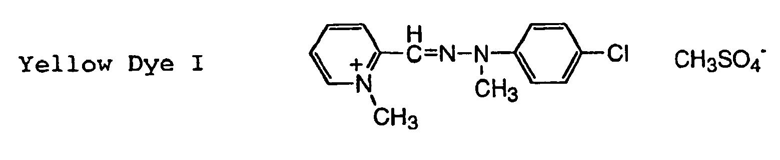

The cationic direct dyes (1) to be incorporated in the first part as the component (C) may be used either singly or in combination. Its (their) content is preferably 0.001 to 10 wt.%, especially 0.01 to 5 wt.% based on the whole composition.

Examples of the alkali agent to be incorporated in the first component part as component (D) include alkanolamines such as monoethanolamine and isopropanolamine, guanidium salts such as guanidine carbonate and ammonia. These alkali agents may be used either singly or in combination. Its (their) content, is preferably 0.1 to 10 wt.%, more preferably 0.5 to 5 wt.%, especially 1 to 3 wt.%, each based on the whole composition. The first component part has a pH of 8 to 12, but that of 8.5 to 11 is especially preferred.

As the reducing agent to be incorporated in the first component part as component (E), those ordinarily employed for a hair coloring composition may be used. Sulfites, thioglycolic acid and ascorbic acid can be mentioned as preferred examples. These reducing agents may be used either singly or in combination. Its (their) content is preferably 0.1 to 5 wt.%, especially 0.3 to 3 wt.% based on the whole composition.

Water as component (F) is added as needed to not only the first component part but also the second component part. Its content is preferably 20 wt.% or greater based on the whole composition.

Examples of the oxidizing agent to be incorporated in the second component part include hydrogen peroxide, urea peroxide, bromates of an alkali metal and peracid salts (perbromates, persulfates and perborates) of an alkali metal. Of these, hydrogen peroxide is particularly preferred. The oxidizing agent, for example, hydrogen peroxide is preferably added in an amount ranging from 1 to 12 wt.% from the viewpoints of sufficient bleaching and hair dyeing effects and low irritation to the scalp.

In addition to the above-described components, those ordinarily used in the field of cosmetics can be incorporated in the hair dye of the present invention. Examples of such an optional component include natural or synthetic polymer compounds, oils and fats, hydrocarbons, higher alcohols, monohydric or polyhydric alcohols, silicone derivatives, amine oxide, amino acid derivatives, protein derivatives, antiseptics, sequestering agents, antioxidants, stabilizers for hydrogen peroxide, plant extracts, vitamins, pigments, ultraviolet absorbers, perfumes and pH regulators.

The hair dye of the invention is used by mixing the first component part with the second component part upon usage and applying the mixture to the hair. A mixing ratio ranging from 2:1 to 1:3 (weight ratio) is preferred. These first and second component parts can each be formed into a liquid, emulsion, cream, gel, paste or mousse in a conventional manner. They can also be formed into an aerosol.

The hair dye of the present invention in the using stage preferably has a pH ranging from 8 to 12, especially 8.5 to 11. At a pH less than 8, effects of the present invention are not available sufficiently, while at a pH exceeding 12, the resulting hair dye causes a severe scalp irritation and is therefore unsuited for practical use.

### -Examples-

### Example 1 and Comparative Examples 1,2

Hair dyes as shown in Table 1 were prepared and their storage stability and color fastness were evaluated.

### (Storage stability)

### Evaluation method

The first and second component parts of the hair dye, each immediately after preparation, were mixed at a weight ratio of 1:1. The mixture (1 g) was then applied uniformly to a hair bundle (having a length of 10 cm and weight of 1 g) of goat hair. After the hair bundle was allowed to stand for 20 minutes in a thermostat of 30°C, it was rinsed with warm water of 40°C. After treatment with a shampoo and a rinse and drying, it was stored in a storage cabinet of -5°C. In a similar manner, another hair bundle of goat hair was treated with the above-described hair dye stored for 3 months in a thermostat of 40°C.

These two hair bundles were compared by a panel of 50 experts and evaluated whether "they have almost a similar color shade", or "they are different in a color shade". Evaluation criteria

A: at least 90% of the experts answered, "they have almost a similar color shade".

B: at least 70% but less than 90% of the experts answered, "they have almost a similar color shade".

C: at least 20% but less than 70% of the experts answered, "they have almost a similar color shade".

D: less than 20% of the experts answered, "they have almost a similar color shade".

### (Color fastness)

### Evaluation method

A difference in the color shade between the goat hair dyed with the above-described hair dye stored for 3 months and the thus-dyed goat hair further subjected to three times repetition of a series of shampooing, rinsing and drying was evaluated by a panel of 50 experts whether "they have almost a similar color shade" or "they are different in a color shade".

### Evaluation criteria

A: at least 80% of the experts answered, "they have almost a similar color shade".
B: at least 60% but less than 80% of the experts answered, "they have almost a similar color shade".
C: at least 20% but less than 60% of the experts answered, "they have almost a similar color shade".
D: less than 20% of the experts answered, "they have almost a similar color shade".

**Table 1**

| (wt.%) | | Ex. | Comp. Ex. | |
|---|---|---|---|---|
| | | 1 | 1 | 2 |
| First component part | (A): Toluene-2,5-diamine | 1 | | |
| | (B): 2,4-Diaminophenoxyethanol | 1.37 | | |
| | (C): Yellow dye A | 0.3 | | |
| | 2-Amino-5-nitrophenol | | 0.3 | |
| | Basic Yellow 57 | | | 0.3 |
| | 28 wt.% Aqueous ammonia | | 5 | |
| | Monoethanolamine | 2 | | |
| | Cetanol | 8.5 | | |
| | Polyoxyethylene (40) cetyl ether | 3 | | |
| | Polyoxyethylene (2) cetyl ether | 3.5 | | |
| | Stearyl trimethyl ammonium chloride | 2 | | |
| | Liquid paraffin | 0.5 | | |
| | Sodium sulfite | 0.5 | | |
| | Ascorbic acid | 0.5 | | |
| | Sodium edetate | 0.1 | | |
| | Perfume | q.s. | | |
| | Ammonium chloride Ammonium chloride | Amount to adjust pH to 10 | | |
| | Water | Balance | | |
| Second component part | 35 wt.% Aqueous hydrogen peroxide | 17.1 | | |
| | Methylparaben | 0.1 | | |
| | Phosphoric acid | Amount to adjust pH 3.5 | | |
| | Water | Balance | | |
| Evaluation | Dyeing test after storage | A | A | D |
| | Color fastness test | A | D | - |

### Examples 2 to 5

In a similar manner to Example 1 except that the below-described components (A), (B) and (C) were used as the precursor, coupler and cationic dye (1) in the first component part, respectively, dyes were prepared (the first and second component parts were mixed at an equivalent weight ratio). Each of them was found to have excellent color fastness and storage stability.

### (Example 2)

| | | |
|---|---|---|
| (A) | Para-phenylenediamine | 1.5 wt.% |
| (B) | Meta-aminophenol | 1.5 |
| (C) | Yellow dye B | 0.5 |

The resulting hair dye is capable of dyeing the hair in a yellowish brown color.

### (Example 3)

| | | |
|---|---|---|
| (A) | Para-aminophenol | 0.9 wt.% |
| (B) | Para-aminoorthocresol | 1 |
| (C) | Yellow dye C | 0.5 |

The resulting hair dye is capable of dyeing the hair in a bright orange color.

### (Example 4)

| | | |
|---|---|---|
| (A) | 2-(2-Hydroxyethyl)-p-phenylenediamine | 2.2 wt.% |
| (B) | 5-(2-Hydroxyethyl)amino-2-methylphenol | 2 |
| (C) | Yellow dye A | 0.4 |

The resulting hair dye is capable of dyeing the hair in an orange color.

### (Example 5)

| | | |
|---|---|---|
| (A) | N,N-Bis(2-hydroxyethyl)-p-phenylenediamine | 2.1 wt.% |
| (B) | 2,4-Diaminophenoxyethanol | 1.8 |
| (C) | Yellow dye D | 0.3 |

The resulting hair dye is capable of dyeing the hair in a bluish green color.

## Claims

1. A hair dye comprising a first component part containing an oxidation dye precursor and a second component part containing an oxidizing agent, wherein the first part contains (A) a precursor, (B) a meta-diaminobenzene, meta-aminophenol, pyridine or naphthol type coupler, (C) a cationic direct dye represented by the following formula (1): 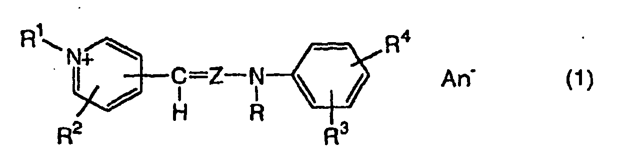 [wherein, R¹ represents a C₁₋₄ alkyl group which may have a hydroxy or cyano group as a substituent, R² represents a hydrogen atom, a C₁₋₄ alkyl group or a cyano group, Z represents a nitrogen atom or a methine group, R represents a hydrogen atom or a C₁₋₄ alkyl group, R³ and R⁴ each independently represents a hydrogen atom, a C₁₋₄ alkyl group, C₁₋₄ alkoxy group, an amino group or a halogen atom, or R and R³ may form a 5- or 6-membered ring together with the adjacent nitrogen atom and the carbon atom on the benzene ring, and An⁻ represents an anion], (D) an alkali agent, (E) a reducing agent and (F) water; and has a pH of 8 to 12.

2. A hair dyeing method comprising mixing first and second component parts of a hair dye as claimed in claim 1 and then applying the mixture to the hair.

## Patentansprüche

1. Haarfarbstoff, umfassend einen ersten Komponententeil, umfassend einen Oxidationsfarbstoffvorläufer, und einen zweiten Komponententeil, umfassend ein Oxidationsmittel, worin der erste Teil (A) einen Vorläufer, (B) einen Kuppler vom meta-Diaminobenzol-, meta-Aminophenol-, Pyridin- oder Naphtholtyp, (C) einen kationischen direkt ziehenden Farbstoff mit der Formel (1): 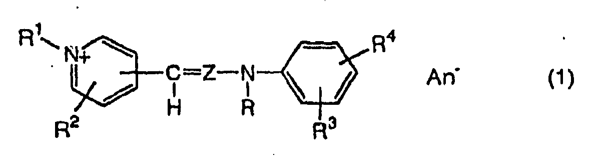 [worin R¹ eine C₁₋₄-Alkylgruppe ist, die eine Hydroxy- oder Cyanogruppe als Substituenten aufweisen kann, R² ein Wasserstoffatom, eine C₁₋₄-Alkylgruppe oder Cyanogruppe ist, Z ein Stickstoffatom oder eine Methingruppe ist, R ein Wasserstoffatom oder eine C₁₋₄-Alkylgruppe ist, R³ und R⁴ jeweils unabhängig ein Wasserstoffatom, eine C₁₋₄-Alkylgruppe, C₁₋₄-Alkoxygruppe, Aminogruppe oder Halogenatom sind oder R und R³ einen 5- oder 6-gliedrigen Ring zusammen mit dem benachbarten Stickstoffatom und dem Kohlenstoffatom am Benzolring bilden können und An⁻ ein Anion ist], (D) ein Alkalimittel, (E) ein Reduktionsmittel und (F) Wasser enthält; und einen pH von 8 bis 12 aufweist.

2. Haarfärbeverfahren, umfassend das Mischen des ersten und des zweiten Komponententeils eines Haarfarbstoffes gemäß Anspruch 1 und das anschließende Auftragen der Mischung auf das Haar.

## Revendications

1. Colorant pour cheveux comprenant une première partie de constituants contenant un précurseur de colorant d'oxydation et une deuxième partie de constituants contenant un agent d'oxydation, dans lequel la première partie contient (A) un précurseur, (B) un coupleur de type méta-diaminobenzène, méta-aminophénol, pyridine ou naphtol, (C) un colorant direct cationique représenté par la formule (1) ci-dessous: 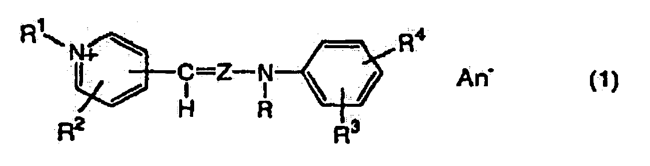 [dans laquelle R¹ représente un groupe alkyle en C₁-C₄ qui peut avoir un groupe hydroxy ou cyano comme substituant, R² représente un atome d'hydrogène, un groupe alkyle en C₁-C₄ ou un groupe cyano, Z représente un atome d'azote ou un groupe méthine, R représente un atome d'hydrogène ou un groupe alkyle en C₁-C₄, R³ et R⁴ représentent chacun indépendamment un atome d'hydrogène, un groupe alkyle en C₁-C₄, un groupe alcoxy en C₁-C₄, un groupe amino ou un atome d'halogène, ou R et R³ peuvent former un cycle de 5 ou 6 chaînons avec l'atome d'azote adjacent et l'atome de carbone du cycle benzène, et An⁻ représente un anion], (D) un agent alcalin, (E) un agent réducteur et (F) de l'eau; et a un pH de 8 à 12.

2. Procédé de coloration des cheveux comprenant le mélange des première et deuxième parties de constituants d'un colorant pour cheveux selon la revendication 1, puis l'application du mélange sur les cheveux.
